# EUROPEAN PATENT APPLICATION

(11) **EP 4 785 975 A1**
(43) Date of publication of application: **05.08.2026**
(21) Application number: 26155620.3
(22) Date of filing: 02.02.2026
(51) Int. Cl.: A61M 5/14, A61M 5/168, G16H 20/17, G16H 40/63

(54) **INTRAVENOUS INFUSION SYSTEM AND VISUALIZER**

(30) Priority: 04.02.2025 US 202563753547 P
(71) Applicant: Fresenius Kabi USA, LLC, Lake Zurich, IL 60047 (US)
(72) Inventor: POWERS, Benjamin G., Lake Zurich, 60047 (US)
(74) Representative: Maikowski & Ninnemann Patentanwälte Partnerschaft mbB

(57) **Abstract**

An intravenous infusion system includes a plurality of infusion pumps each configured to convey a fluid to a subject during an intravenous infusion procedure, a controller programmed to receive information regarding the pumps, and a display screen configured to receive input from the controller and to display a visual representation of the pumps based at least in part on the input from the controller. The controller may be programmed to determine whether there is a possible negative interaction between operation of at least two of the pumps during the intravenous infusion procedure and to control the display screen to display a notice regarding the negative interaction upon determining that the negative interaction is possible. The controller and display screen may also or alternatively cooperate to determine and display information regarding the locations of fluids during the procedure and/or to determine and display a total fluid intake level.

## Description

### Background

### Field of the Disclosure

The present subject matter relates to intravenous infusion systems. More particularly, the present subject matter relates to intravenous infusion systems including multiple pumps and/or fluids to be delivered to a subject and a display screen for visual representation of the pumps and fluids during an intravenous infusion procedure.

### Description of Related Art

During a medical procedure, it is frequently necessary to infuse a fluid, such as a medicament, into the vascular system of a subject (such as a patient or a blood donor). Fluids may be infused via a pump or via gravity or may be infused manually, such as via a syringe operated by a technician.

During certain medical procedures, it is necessary to infuse multiple fluids into a subject. When multiple fluids are to be infused into a subject during a procedure (whether simultaneously or sequentially), it is important to be mindful of various aspects of the procedure so as to avoid negative interactions. For example, it is important to be mindful of compositions of the fluids being infused into the patient, as it may be the case that two fluids are incompatible with each other in some way, such as one reducing the effectiveness of the other. It may also be important to be mindful of the total fluid load (i.e., the amount of fluid administered to a subject during a procedure) so as to avoid hypervolemia or fluid overload. When multiple pumps are employed to convey fluids to a subject, it may be important to ensure that infusion of one fluid does not unintentionally cause another fluid in a fluid line to be bolused to the subject.

Even when one is mindful of these considerations, it may be difficult for an operator or physician to accurately assess the various risks due to a lack of information, such as an incomplete understanding of how tubing characteristics (such as diameter and length), can affect various aspects of fluid delivery, such as time-to-occlusion, delay in delivering an infusate to a subject, hydro-dynamic backpressure, drug mixing, etc. Further, even when the risks are properly assessed at the beginning of a procedure, they may change during the course of a procedure in ways that cannot be predicted by and accounted for by the operator or physician.

Accordingly, it would be advantageous to provide a system allowing for improved assessment, visualization, and control of various aspects of an intravenous infusion procedure.

### Summary

There are several aspects of the present subject matter which may be embodied separately or together in the devices and methods described and claimed below. These aspects may be employed alone or in combination with other aspects of the subject matter described herein, and the description of these aspects together is not intended to preclude the use of these aspects separately or the claiming of such aspects separately as set forth in the claims appended hereto. The controller is further programmed to determine whether there is a possible negative interaction between operation of at least two of the infusion pumps during the intravenous infusion procedure. Upon determining that the negative interaction is possible, the controller controls the display screen to display a notice regarding the negative interaction; otherwise, the controller controls the display screen to not display the notice.

In one aspect, an intravenous infusion system includes a plurality of infusion pumps each configured to convey a fluid to a subject during an intravenous infusion procedure, a controller programmed to receive information regarding each one of the infusion pumps, and a display screen configured to receive input from the controller and to display a visual representation of each one of the infusion pumps based at least in part on the input from the controller. The controller is further programmed to control the controller to display information regarding locations of the fluids during the intravenous infusion procedure.

In another aspect, an intravenous infusion system includes a plurality of infusion pumps each configured to convey a fluid to a subject during an intravenous infusion procedure, a controller programmed to receive information regarding each one of the infusion pumps, and a display screen configured to receive input from the controller and to display a visual representation of each one of the infusion pumps based at least in part on the input from the controller.

In yet another aspect, an intravenous infusion system includes a plurality of infusion pumps each configured to convey a fluid to a subject during an intravenous infusion procedure, a controller programmed to receive information regarding each one of the infusion pumps, and a display screen configured to receive input from the controller and to display a visual representation of each one of the infusion pumps based at least in part on the input from the controller. The controller is further programmed to determine a total fluid intake level and to control the display screen to display the total fluid intake level.

### Brief Description of the Drawings

Fig. 1 is a front elevational view of an intravenous infusion system according to an aspect of the present disclosure;
Fig. 2 is a front perspective view of another embodiment of an intravenous infusion system according to an aspect of the present disclosure;
Fig. 3 illustrates an exemplary image appearing on a display screen of the intravenous infusion system of Figs. 1 or 2, showing an infusion pump being added to the system;
Fig. 4 illustrates an exemplary image appearing on a display screen of the intravenous infusion system of Figs. 1 or 2, showing an access site being selected;
Fig. 5 illustrates an exemplary image appearing on a display screen of the intravenous infusion system of Figs. 1 or 2, showing a type of catheter of an infusion set being selected;
Fig. 6 illustrates an exemplary image appearing on a display screen of the intravenous infusion system of Figs. 1 or 2, showing a number of lumens of an infusion set being selected;
Fig. 7 illustrates an exemplary image appearing on a display screen of the intravenous infusion system of Figs. 1 or 2, showing a portion of an infusion set being added to the image;
Fig. 8 illustrates an exemplary image appearing on a display screen of the intravenous infusion system of Figs. 1 or 2, showing a connection type for an infusion set being selected;
Fig. 9 illustrates an exemplary image appearing on a display screen of the intravenous infusion system of Figs. 1 or 2, showing additional characteristics of an infusion set being selected;
Fig. 10 illustrates an exemplary image appearing on a display screen of the intravenous infusion system of Figs. 1 or 2, showing an infusion set being linked to a pump;
Fig. 11 illustrates an exemplary image appearing on a display screen of the intravenous infusion system of Figs. 1 or 2, showing a total fluid intake icon being added to the image;
Fig. 12 illustrates an exemplary image appearing on a display screen of the intravenous infusion system of Figs. 1 or 2, showing an access site being selected for a second infusion;
Fig. 13 illustrates an exemplary image appearing on a display screen of the intravenous infusion system of Figs. 1 or 2, showing a second infusion being added to the image;
Fig. 14 illustrates an exemplary image appearing on a display screen of the intravenous infusion system of Figs. 1 or 2, showing a fluid intake notice being added to the image;
Fig. 15 illustrates an exemplary image appearing on a display screen of the intravenous infusion system of Figs. 1 or 2, showing an adjustment being made to the flow rate of the second infusion of Fig. 13;
Figs. 16-18 illustrate exemplary images appearing on a display screen of the intravenous infusion system of Figs. 1 or 2, showing a third infusion being added to the image;
Fig. 19 illustrates an exemplary image appearing on a display screen of the intravenous infusion system of Figs. 1 or 2, showing a backpressure notice being added to the image;
Fig. 20 illustrates an exemplary image appearing on a display screen of the intravenous infusion system of Figs. 1 or 2, showing an alternative arrangement for a third infusion being added to the image;
Fig. 21 illustrates an exemplary image appearing on a display screen of the intravenous infusion system of Figs. 1 or 2, showing an occlusion notice being added to the image;
Fig. 22 illustrates an exemplary image appearing on a display screen of the intravenous infusion system of Figs. 1 or 2, showing yet another alternative arrangement for a third infusion being added to the image; and
Figs. 23-33 illustrate exemplary images appearing on a display screen of the intravenous infusion system of Figs. 1 or 2, showing the positions of various fluids at different times during an intravenous infusion procedure.

### Description of the Illustrated Embodiments

The embodiments disclosed herein are for the purpose of providing an exemplary description of the present subject matter. They are, however, only exemplary, and the present subject matter may be embodied in various forms. Therefore, specific details disclosed herein are not to be interpreted as limiting the subject matter as defined in the accompanying claims.

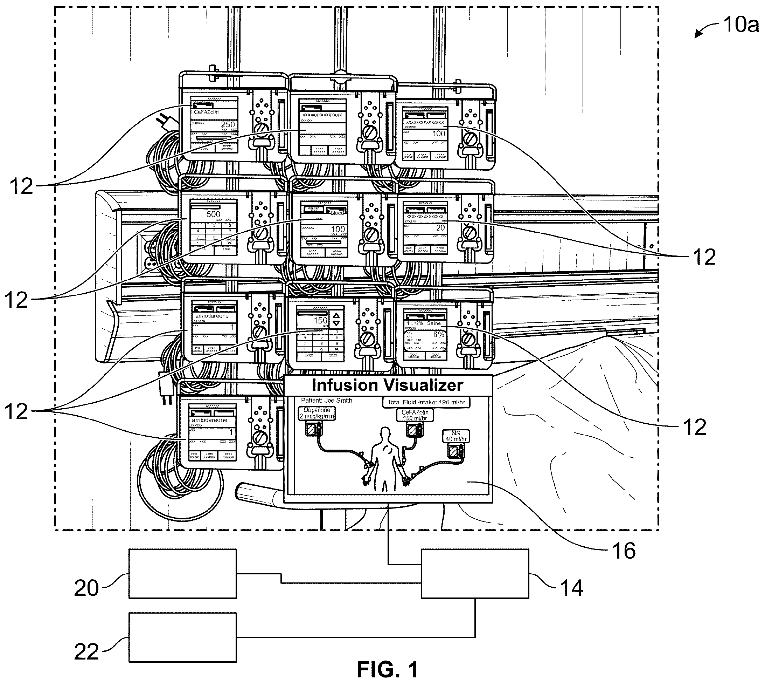

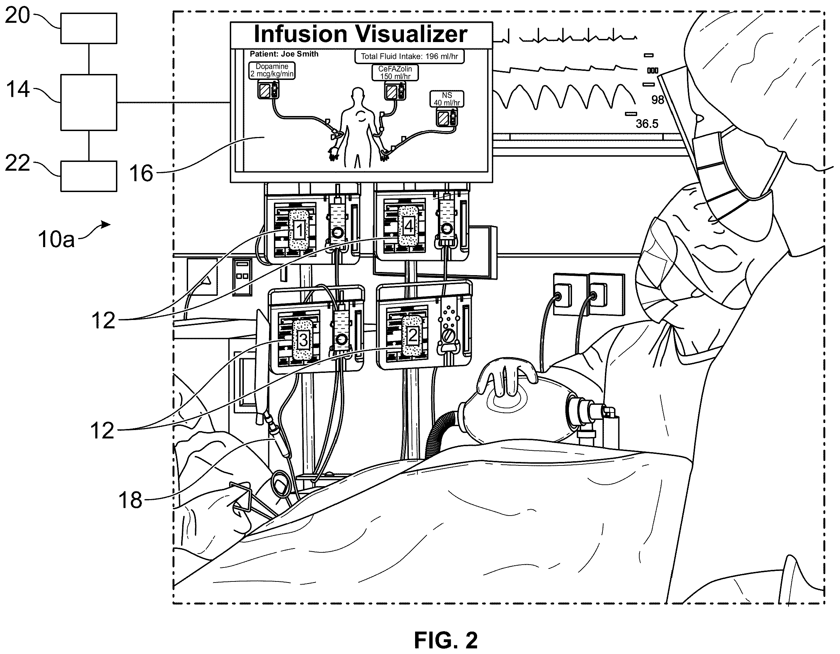

Figs. 1 and 2 illustrate exemplary embodiments of intravenous infusion systems 10a and 10b (collectively referred to herein using the reference numeral 10) according to the present disclosure. The intravenous infusion system 10a of Fig. 1 is illustrated adjacent to a bed of a patient or subject, while the intravenous infusion system 10b of Fig. 2 is illustrated in an operating room. In other embodiments, the intravenous infusion system 10 or individual components thereof may be positioned in any other environment (e.g., a nurse's central station) without departing from the scope of the present disclosure.

An intravenous infusion system 10 according to the present disclosure includes at least one infusion pump 12, but more typically a plurality of infusion pumps 12 (as shown in Figs. 1 and 2), which may be identically configured or may be differently configured. In the illustrated embodiments, each pump 12 is configured as a large volume pump, though it should be understood that the pumps 12 of an infusion system 10 according to the present disclosure may be otherwise configured without departing from the scope of the present disclosure. Furthermore, while Fig. 1 illustrates an intravenous infusion system 10a employing ten pumps 12 and Fig. 2 illustrates a system 10b employing four pumps 12, it should be understood that an intravenous infusion system 10 according to the present disclosure may include any number of pumps 12 without departing from the scope of the present disclosure. Finally, it should be understood that an intravenous infusion system 10 according to the present disclosure may include more pumps 12 than are required for execution of a particular intravenous infusion procedure, with any excess pumps 12 simply remaining inactive during the procedure.

As used herein, the term "intravenous infusion procedure" is intended to encompass any medical procedure in which two or more infusion pumps are employed to intravenously deliver one or more fluids to a subject. This may include procedures involving only the delivery of one or more fluids to a subject and procedures including additional operations, such as procedures in which blood is drawn from a patient and one or more fluids (such as a separated blood component and a replacement fluid) are conveyed to the patient. The term is also intended to encompass medical procedures in which a single infusion pump is used to intravenously deliver two or more different fluids to a subject.

In addition to one or more pumps 12, intravenous infusion systems 10 according to the present disclosure include a controller 14 and a display screen 16. The controller 14 is programmed to receive information regarding each one of the infusion pumps 12, which may include the controller 14 receiving information directly from a pump 12 (via a wireless or wired connection) or from an operator or from some other source. The controller 14 may be further programmed to control operation of the pumps 12, which may include the controller 14 directly commanding the pumps 12 to operate in a particular manner or the controller 14 communicating with an intermediary (e.g., a dedicated pump controller) that issues commands to the pumps 12.

The controller 14 may be incorporated into the display screen 16 or may be separately provided and may be variously configured without departing from the scope of the present disclosure (provided that it has the functionality described herein). In one embodiment, the controller 14 may include a microprocessor (which, in fact may include multiple physical and/or virtual processors). According to other embodiments, the controller 14 may include one or more electrical circuits designed to carry out the actions described herein. In fact, the controller 14 may include a microprocessor and other circuits or circuitry. In addition, the controller 14 may include one or more memories. The instructions by which the microprocessor is programmed may be stored on the memory associated with the microprocessor, which memory/memories may include one or more tangible non-transitory computer readable memories, having computer executable instructions stored thereon, which when executed by the microprocessor, may cause the microprocessor to carry out one or more actions as described herein. In one exemplary embodiment, the controller 14 comprises a main processing unit (MPU), which can comprise, e.g., a PENTIUM^{®} type microprocessor made by Intel Corporation, although other types of conventional microprocessors can be used.

In addition to receiving information regarding the infusion pumps 12, the controller 14 is further programmed to transmit input to the display screen 16, with the display screen 16 displaying a visual representation of the pumps 12, along with additional information regarding an intravenous infusion procedure being executed or to be executed by the system 10 (as will be described in greater detail). In one embodiment, the display screen 16 is configured to have only visual (and, optionally, audio) output functionality (along with being configured to receive input from the controller 14). In other embodiments, the display screen 16 may be configured to additionally receive input and/or information from other sources (such as an operator) and to provide output (whether in the form of commands or information) to the controller 14. If the display screen 16 is configured to receive input from an operator, it may be configured as a touch screen and/or have an associated entry device (such as a keyboard and/or mouse).

The intravenous infusion system 10 also includes infusion sets 18 (Fig. 2) that are configured to place the pumps 12 in fluidic communication with the vasculature of a subject. The configuration of the infusion sets 18 may vary without departing from the scope of the present disclosure, with the configuration typically depending in part on the configuration of the associated pump(s) 12. The infusion sets 18 will include a conduit (e.g., a tube) to allow for the flow of fluid (under action of a pump 12) from a source (such as a container) to the vasculature of a subject, but may include additional components (such as valves or drip chambers or cannulas) without departing from the scope of the present disclosure. As will be explained in greater detail, on account of a wide variety of infusion sets 18 being suitable for use with intravenous infusion systems 10 according to the present disclosure, it may be advantageous for the controller 14 to be informed of the configuration of the infusion set 18 associated with each active pump 12, with the controller 14 accounting for the configurations of the infusion sets 18 being employed in an intravenous infusion procedure in its analysis of the procedure.

The intravenous infusion system 10 may include additional components without departing from the scope of the present disclosure. For example, the system 10 may include supports (e.g., poles) for the infusion pumps 12 and/or supports (e.g., hooks or hangers) for fluid containers and/or wheels or rollers for moving the system 10 from one location to another. In another embodiment, the system 10 may include additional output devices, such as speakers, lights, and/or one or more additional display screens (rather than having only a single screen) and/or input devices, such as an RFID reader or barcode scanner 20. In yet another example, the system 10 may include a pump detector 22 (e.g., a sensor or RFID reader) that automatically detects a pump 12 located in the vicinity of the pump detector 22.

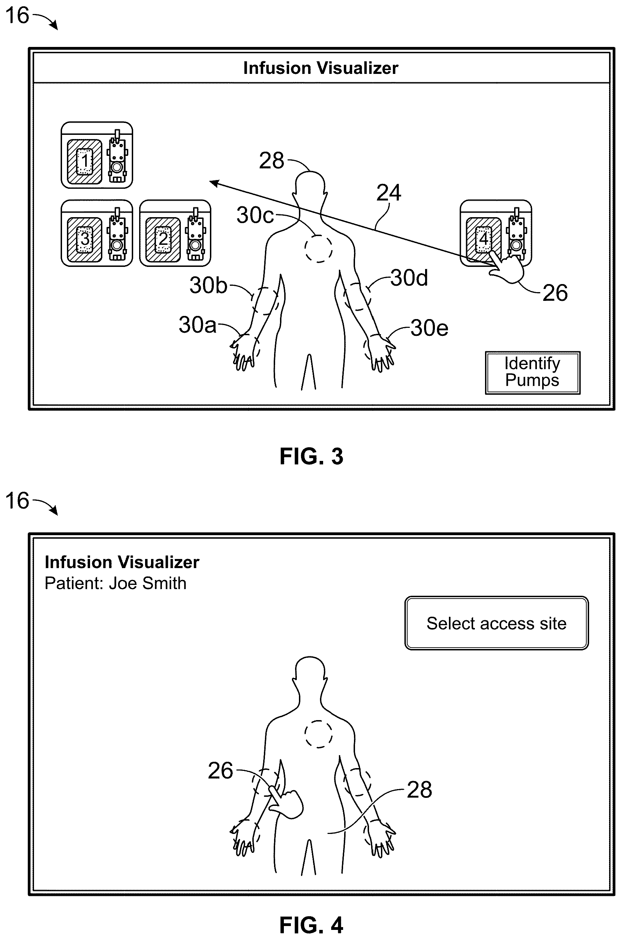

Figs. 3-33 illustrate exemplary images that may be displayed by the display screen 16 at different times before and/or during an intravenous infusion procedure. It should be understood that the illustrated images are merely exemplary and that the same information may be shown on the display screen 16 in any of a number of ways without departing from the scope of the present disclosure.

Fig. 3 shows an image of a pump 12 being added to the system 10 as part of an "identify pumps" step that takes place before fluid delivery to a subject. In the image of Fig. 3, three pumps (identified in Fig. 3 as "1," "2," and "3") are illustrated as already being active, while a fourth pump (identified in Fig. 3 as "4") is illustrated as being available, but inactive. Fig. 3 shows an arrow 24 pointing from the fourth pump toward the other three pumps, indicating that the fourth pump is being added to the system 10. Fig. 3 shows a cursor icon 26 overlaying the fourth pump, with the cursor icon 26 being manipulated by an operator to move the fourth pump from one side of the display screen 16 to the other side in order to indicate to the controller 14 that the fourth pump is to be active during the intravenous infusion procedure. While Fig. 3 shows the fourth pump being added via input received by an operator, it should be understood that a pump may be added to the system 10 in other ways. For example, a pump 12 that is already present in the system 10 may be powered on, which informs the controller 14 that the pump 12 is to be added to the system 10, with the controller 14 informing the display screen 16 that an image of the pump 12 should be shown on the display screen 16. In another example, a pump 12 may be brought into the vicinity of a pump detector 22, with the pump detector 22 detecting the presence of the new pump 12 (e.g., via a Bluetooth or RFID connection or the like) and informing the controller 14, which then transmits an input to the display screen 16 to present an image of the new pump 12. In yet another example, a barcode on a pump 12 may be scanned using a barcode scanner 20 of the system 10 to add the pump 12 to the system 10. In yet another example, a subject is selected on the display screen 16 to associate a pump 12 with the subject. An operator could likewise select a subject on the display screen 16, which would cause any pumps associated with that subject to be shown on the display screen 16, with any new pump that is subsequently associated with the same subject showing up on the display screen 16 upon the pump being added.

In addition to the various pumps, Fig. 3 shows an icon 28 representing the subject of the intravenous infusion procedure. Prior to (or after) adding the pumps 12 to the system 10, the identity of the subject may be provided to the controller 14, with the controller 14 subsequently linking or associating the pumps 12 of the system 10 with the subject. Identifying the subject may include the controller 14 being provided with or accessing additional information regarding the subject, such as their weight and height and any other information that may be relevant to an intravenous infusion procedure (e.g., allergies, diseases, etc.).

Fig. 3 also shows icons 30a-30e (collectively referred to herein using the reference numeral 30) representing possible access sites for fluidically connecting a pump 12 to the vasculature of the subject via an infusion set 18 of the system 10. While Fig. 3 shows five access site icons 30 (identifying the right hand/wrist 30a, right arm 30b, chest 30c, left arm 30d, and left hand/wrist 30e), any number of access site icons 30 may be provided, with the icons 30 identifying any suitable access sites (such as the neck, abdomen, right leg, and left leg). In the exemplary image shown in Fig. 4, an access site is selected using the cursor icon 26. While Fig. 4 shows a cursor icon 26 being employed, an access site may be selected and shown on the display screen 16 using any suitable approach (e.g., via a keyboard associated with the controller 14).

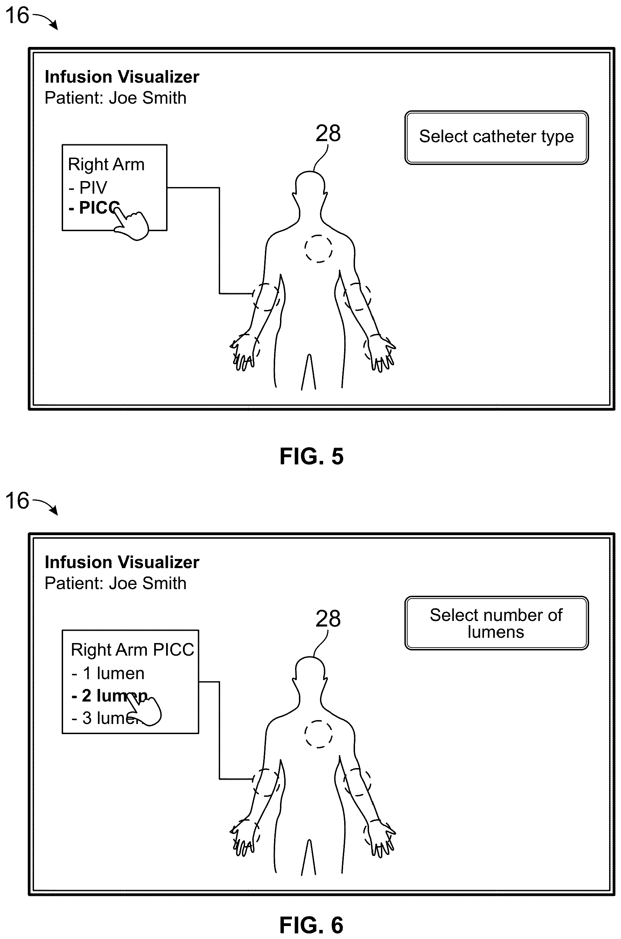

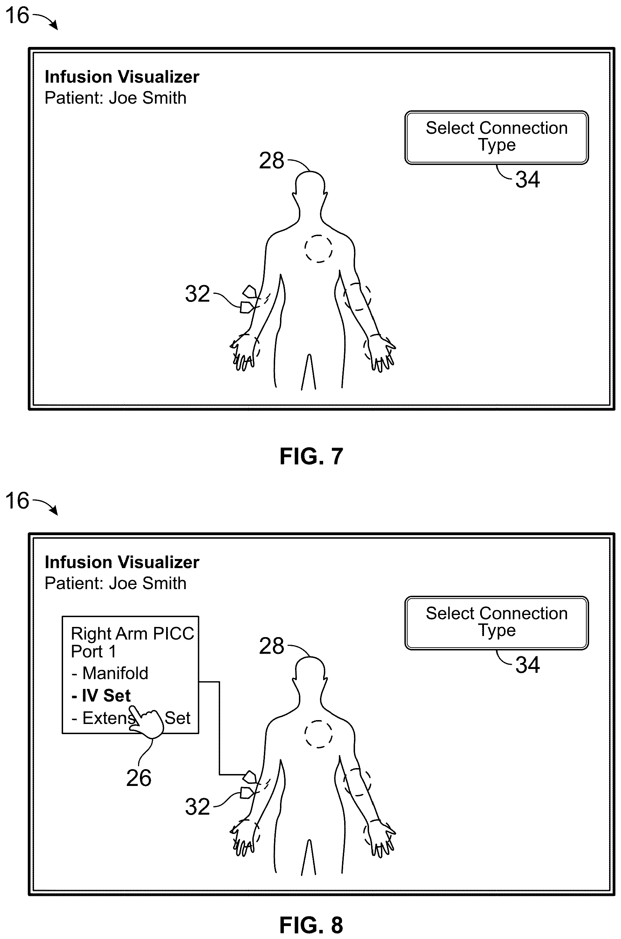

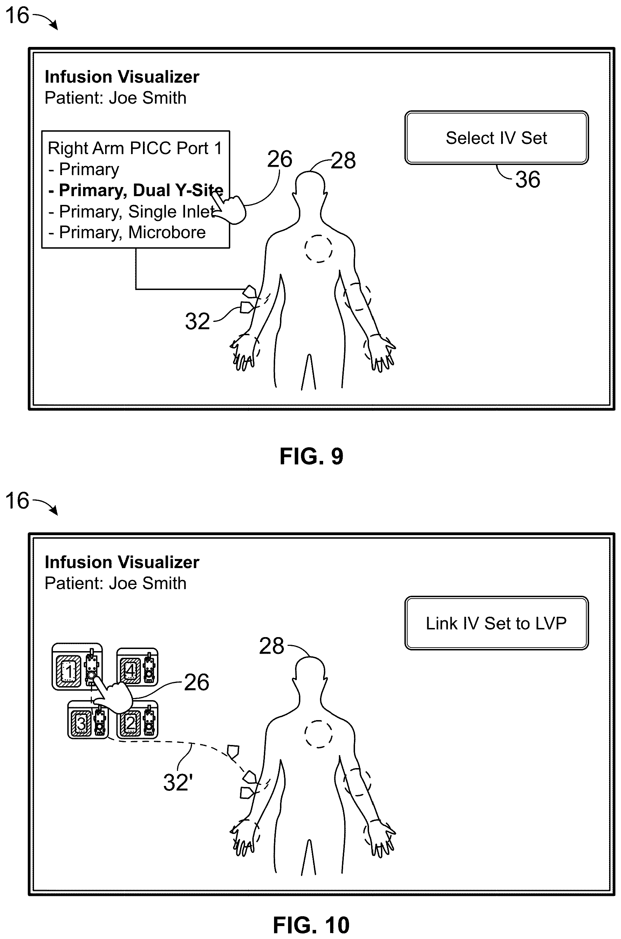

Once an access site has been selected, the configuration of the infusion set 18 associated with that access site is selected, with Figs. 5-9 showing different aspects of an infusion set 18 that may be provided to the controller 14 and then displayed on the display screen 16. In the image of Fig. 5, the type of catheter/needle of the infusion set 18 is selected and shown, with Fig. 5 showing different configurations (peripheral intravenous catheter or peripherally inserted central catheter) that may be selected using the cursor icon 26. In the image of Fig. 6, the number of lumens is selected, with Fig. 6 showing options for one, two, or three lumens. In the image of Fig. 7, an icon 32 representing the type of catheter and number of lumens is shown at the previously selected access site, with the display screen 16 also presenting a "select connection type" icon 34. In the image of Fig. 8, possible connection types (manifold, IV set, and extension set) are shown, with the display screen 16 enabling an operator to select the appropriate connection type using the cursor icon 26. Finally, Fig. 9 shows a "select IV set" icon 36 (after "IV set" was previously selected as the connection type) and different configurations of an IV set (primary; primary, dual Y-site; primary, single inlet; and primary, microbore) that may be selected by an operator using the cursor icon 26. It should be understood that this is only one possible approach for inputting the configuration of an infusion set 18 and that other approaches (e.g., scanning one or more barcodes associated with an infusion set 18 using a barcode scanner 20 of the system 10 or detecting an RFID tag associated with an infusion set 18 using an RFID reader) may be employed without departing from the scope of the present disclosure. It should also be understood that additional information regarding the infusion set 18 (e.g., the diameter and/or length of a conduit) may be provided to the controller 14 and optionally displayed on the display screen 16 without departing from the scope of the present disclosure.

When the configuration of the infusion set 18 associated with the access site has been provided to the controller 14 (according to any suitable approach), an updated infusion set icon 32' may replace the initial infusion set icon 32, as shown in Fig. 10. With the updated infusion set icon 32' shown on the display screen 16, the infusion pump 12 associated with that infusion set 18 may be selected, as also shown in Fig. 10. While Fig. 10 shows the cursor icon 26 being used by an operator to select the appropriate pump, it should be understood that a pump may be linked to an infusion set by any suitable approach, such as scanning a barcode on the pump 12 or, when detailed location data is available, a pump 12 may be automatically associated with the corresponding infusion set and/or access site on the display screen 16, based on its current location. It should also be understood that the visualization sequence described above (with an access site being selected, followed by the details of an infusion set being selected and shown, and then the associated pump being identified) is merely exemplary and that other sequences (e.g., the above sequence in reverse) may be employed without departing from the scope of the present disclosure.

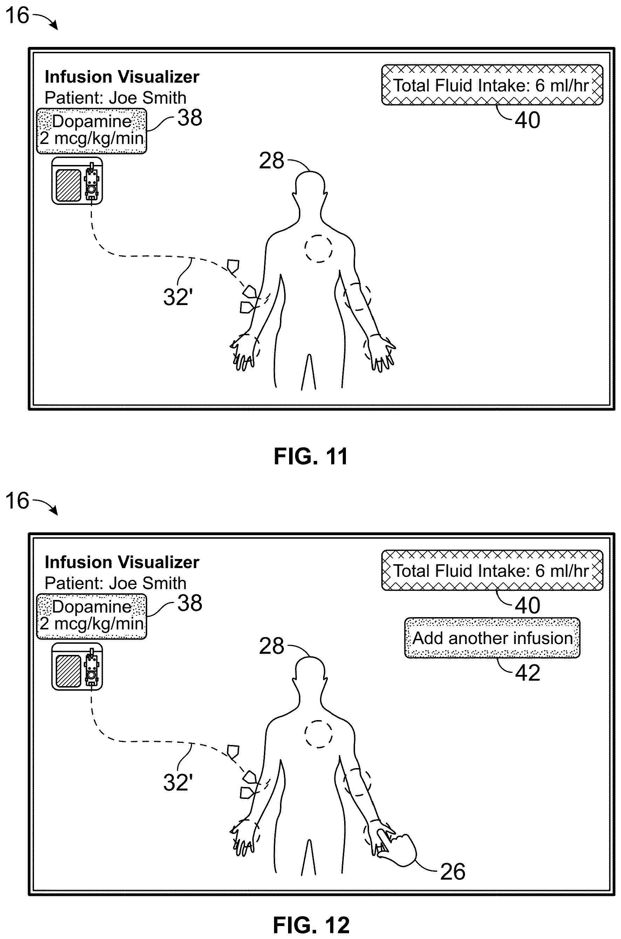

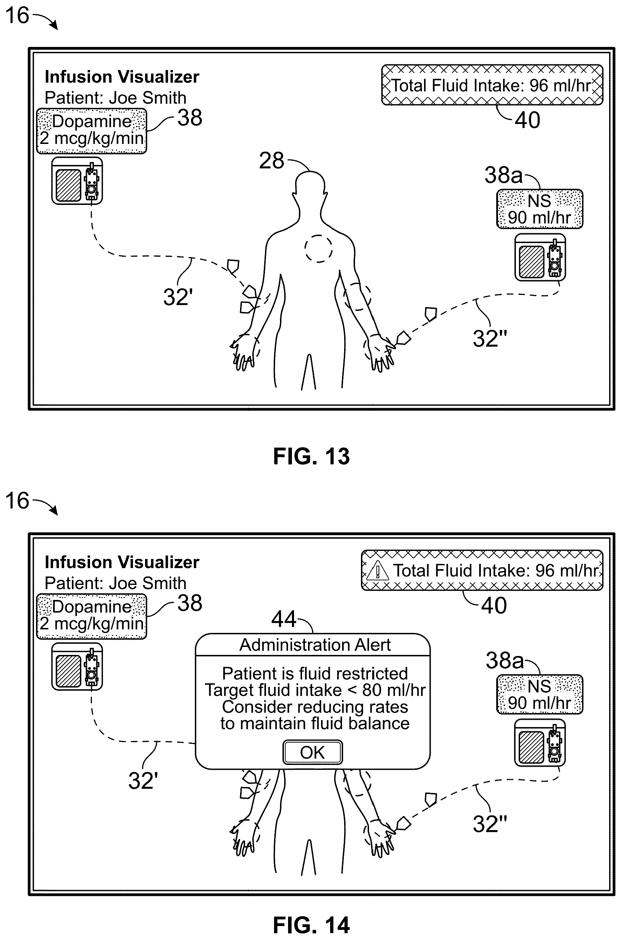

The controller 14 is also informed of the fluid to be delivered to the subject using the pump 12, with that information being provided either before or after the pump 12 has been selected. Fig. 11 shows a fluid icon 38 being shown on the display screen 16, with Fig. 11 also showing a total fluid intake icon 40 being presented on the display screen 16. In the image shown in Fig. 11, only one pump and fluid have been selected, with the value presented in the total fluid intake icon 40 (which has been determined by the controller 14) being the rate at which the selected pump 12 will deliver the selected fluid to the subject. In other embodiments, the total fluid intake icon 40 may display a value corresponding to a total volume of fluid to be delivered to a subject during a procedure or some other value related to the volume of fluid being delivered to a subject during a procedure. In Figs. 12 and 13, the operator is prompted to add another infusion to the procedure (using the icon 42 shown in Fig. 12), with the second infusion being identified in Fig. 13 at 32". It should be understood that the second infusion may be selected using the above-described procedure for identifying the first infusion or may be selected according to any other suitable approach.

With a second infusion being selected (and displayed on the display screen 16), the controller 14 determines the total fluid intake (which, in the illustrated embodiment, accounts for the rate at which fluid is to be delivered to the subject by the first pump during the procedure and the rate at which fluid is to be delivered to the subject by the second pump during the procedure) and provides that input to the display screen 16, which updates the value shown in the total fluid intake icon 40. Upon calculating the total fluid intake, the controller 14 may determine whether the calculated value is appropriate for the subject. The controller 14 may do so by comparing the calculated value to a predetermined or preselected value or to a value that is specific to the subject or by any other suitable approach. If the controller 14 determines that the calculated value is unobjectionable (e.g., if it is not greater than a value that it is compared against), it may proceed without generating a notice; otherwise, if the controller 14 determines that the calculated value is possibly problematic (e.g., if it is greater than a value that it is compared against), it may respond by controlling the display screen 16 to display a fluid intake notice 44 (Fig. 14) regarding the possible negative interaction between operation of the infusion pumps 12. In the illustrated embodiment, the fluid intake notice 44 advises an operator of the possible negative interaction and suggests a possible approach for avoiding the negative interaction, but it should be understood that the fluid intake notice 44 may be differently configured without departing from the scope of the present disclosure.

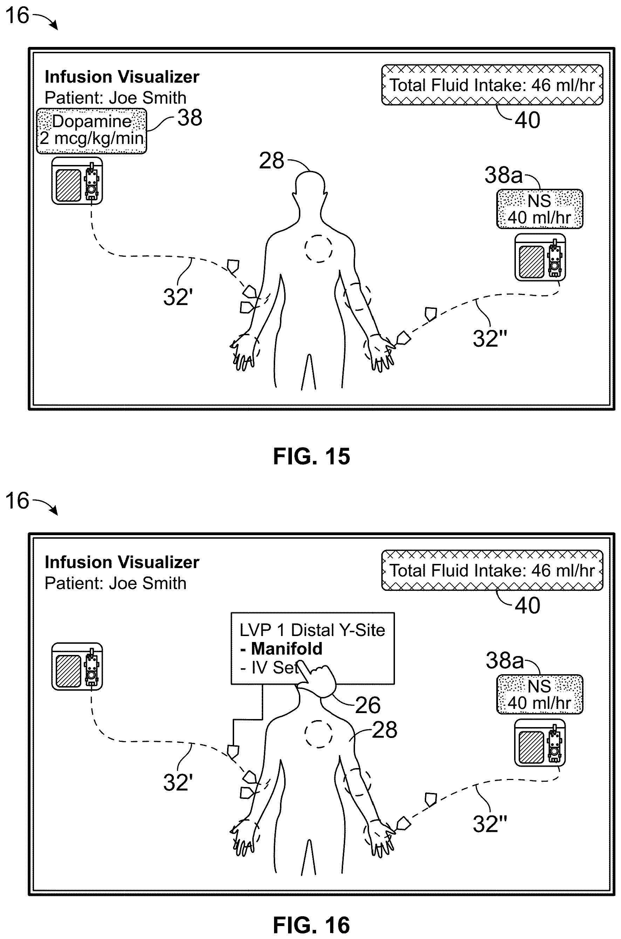

Fig. 15 shows the proposed fluid delivery rate for the second infusion (presented in icon 38a) being adjusted from 90 ml/hr (as shown in Figs. 13 and 14) to 40 ml/hr, which brings the total fluid intake to a calculated value (46 ml/hr) that is less than the value that it was compared against (80 ml/hr). As illustrated in this example, the intravenous infusion system 10 may function to predict a possible negative interaction between the operation of two or more infusion pumps 12 during an intravenous infusion procedure, suggest an approach to avoid the negative interaction, and make an adjustment to the proposed operation of one or more of the infusion pumps 12 (either alone or in combination with an operator) so as to avoid the possible negative interaction. In one embodiment, one pump 12 or infusion may be designated as the "hydration pump" or "hydration infusion," with the fluid delivery rate of that pump 12 or infusion being automatically determined by the controller 14 in view of other infusion rates so as to arrive at a target total fluid intake.

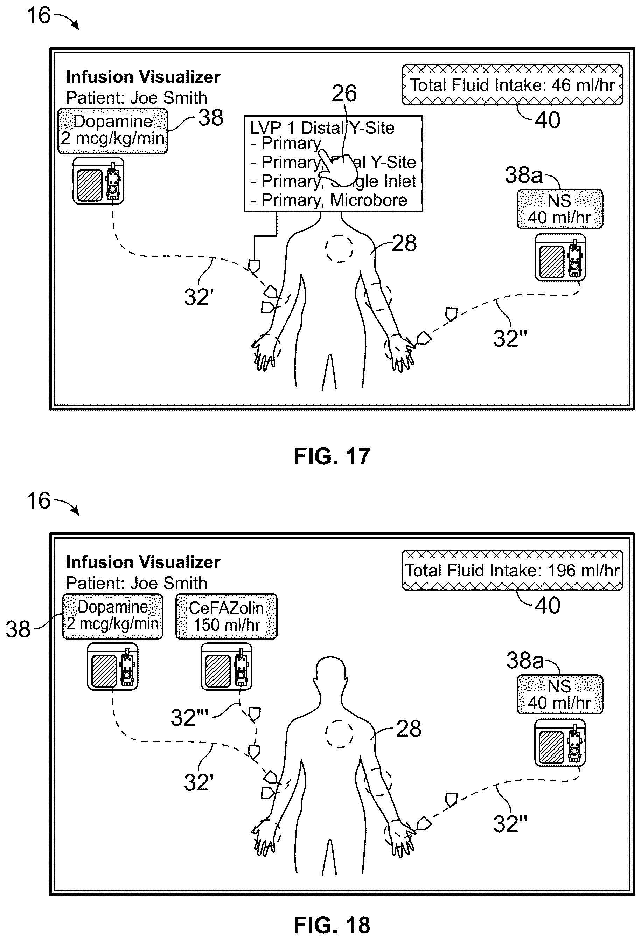

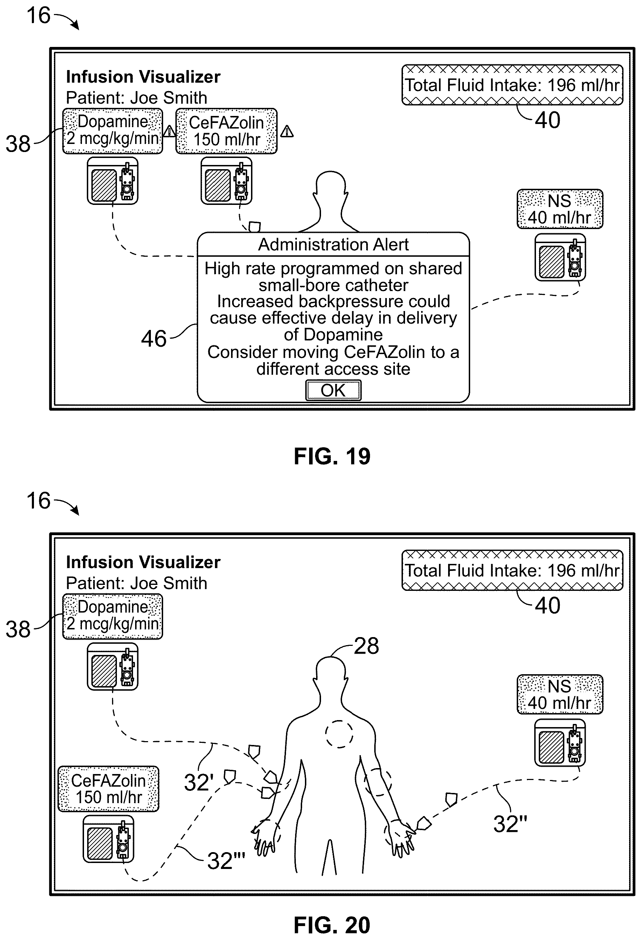

It should be understood that a potentially excessive total fluid intake is not the only negative interaction that may be diagnosed and avoided by the intravenous infusion system 10, with Figs. 16-19 illustrating another example. Figs. 16-18 show a third infusion (represented by icon 32‴ in Fig. 18) being added to the intravenous infusion procedure, in accordance with the approach described above for adding the first infusion to the procedure. As with the second infusion, upon the third infusion being added to the procedure, the controller 14 analyzes the consequences of the new infusion to determine whether any negative interactions are possible. In the illustrated example, the controller 14 determines that adding the third infusion to the same access site as the first infusion could create a negative interaction in which increased backpressure created by the third infusion could delay delivery of fluid in the first infusion. The controller 14 may determine the possibility of an elevated backpressure based on any suitable data, such as (but not limited to) the diameter of a tube or conduit of an infusion set 18, the diameter of an access site, the viscosity of a fluid, and the flow rate of a fluid through a tube or conduit of an infusion set 18. Fig. 19 shows a resulting backpressure notice 46 that is presented by the display screen 16 in response to input provided to it by the controller 14, with the backpressure notice 46 suggesting that the third infusion be moved to a different access site so as to avoid a possible negative interaction. It should be understood that the illustrated backpressure notice 46 is merely exemplary and that such a notice may be differently configured without departing from the scope of the present disclosure.

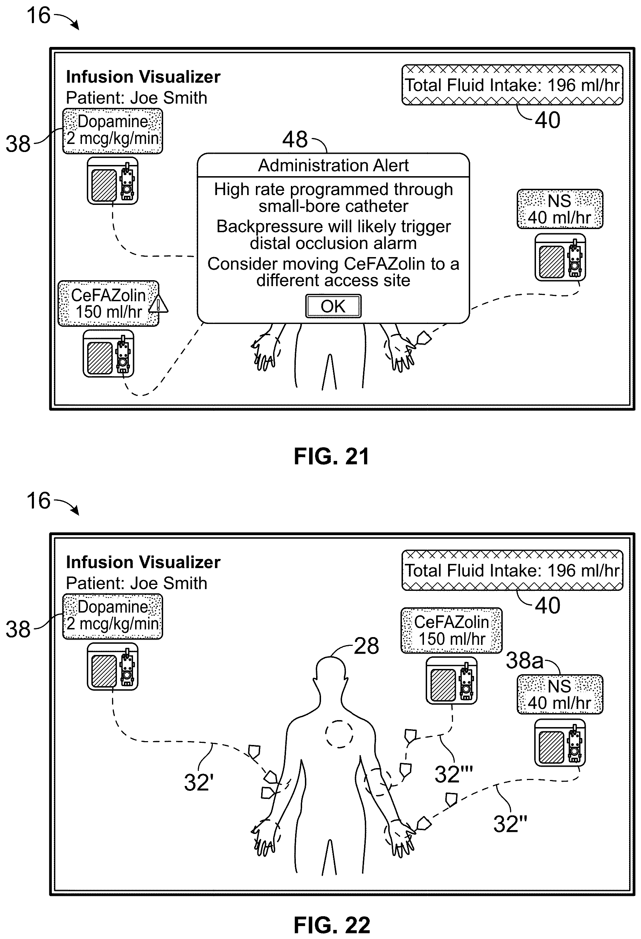

In the illustrated example, in response to the backpressure notice 46 of Fig. 19, the third infusion is moved to a different access site, as shown in Fig. 20. Upon this change being made, the controller 14 again analyzes the consequences of the proposed intravenous infusion procedure to determine whether any negative interactions are possible. In this case, the controller 14 determines that adding the third infusion to a new access site could create a negative interaction in which a high fluid flow rate through a small-bore catheter results in backpressure at a level that would trigger a distal occlusion and, thus, a distal occlusion alarm. The controller 14 may diagnose a possible occlusion risk based on any appropriate data, such as by determining that two pumps 12 having different occlusion alarm settings (e.g., the detected pressure at which an occlusion alarm is generated for the pump 12) are connected through the same port or infusion set 18 and that one of the pumps 12 may stop before the other. Fig. 21 shows a resulting occlusion notice 48 that is presented by the display screen 16 in response to input provided to it by the controller 14, with the occlusion notice 48 suggesting that the third infusion be moved to a different access site so as to avoid a possible negative interaction. It should be understood that the illustrated occlusion notice 48 is merely exemplary and that such a notice may be differently configured without departing from the scope of the present disclosure. For example, the controller 14 may determine the time required for an occlusion to be detected for a particular pump 12 or infusion and, upon determining that the time may be too great, may control the display screen 16 to show an occlusion notice to that effect, along with a suggestion that one or more occlusion alarm settings be modified or that the configuration of the associated infusion set 18 be modified or that the infusion be moved to a dedicated port, etc.

Fig. 22 shows the third infusion having been moved to another access site, with the controller 14 determining that such an intravenous infusion procedure would not be problematic and, thus, not controlling the display screen 16 to show a notice.

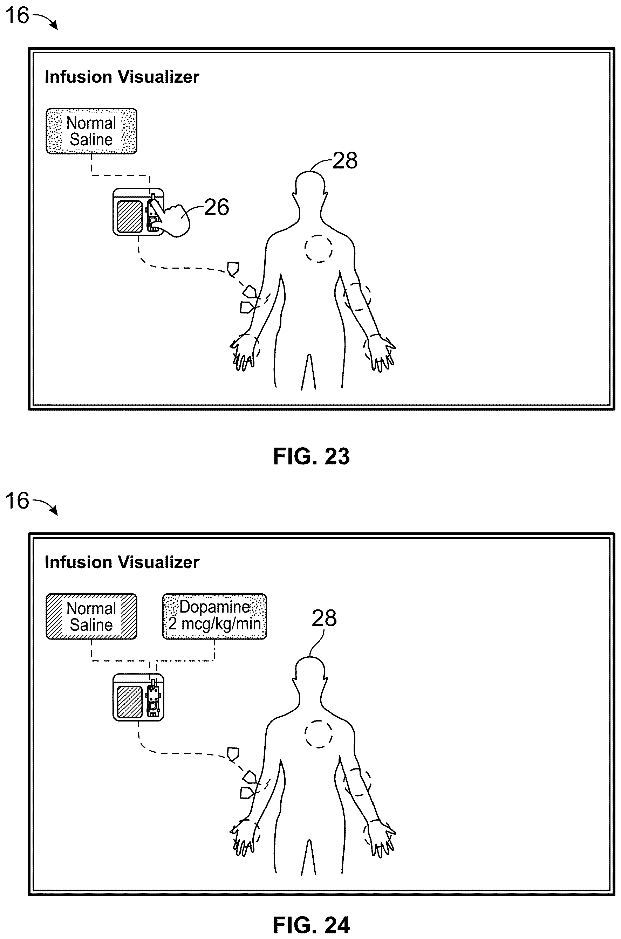

Figs. 23-33 present exemplary images that may be presented on the display screen 16 (based on input from the controller 14) to simulate an intravenous infusion procedure by showing the positions of various fluids at different times during the procedure. Figs. 23 and 24 show a first infusion (Fig. 23) and a second infusion (Fig. 24) being implemented using a single infusion pump. The first infusion provides a first fluid to the subject (with that fluid being identified in Figs. 23 and 24 as a solid line), while the second infusion provides a second fluid to the subject (with that fluid being identified in Fig. 24 as a broken line). It should, thus, be clear from this example that intravenous infusion systems according to the present disclosure are not only advantageous for procedures in which multiple infusion pumps 12 are employed, but in procedures in which multiple fluids are delivered to a subject via a single pump 12.

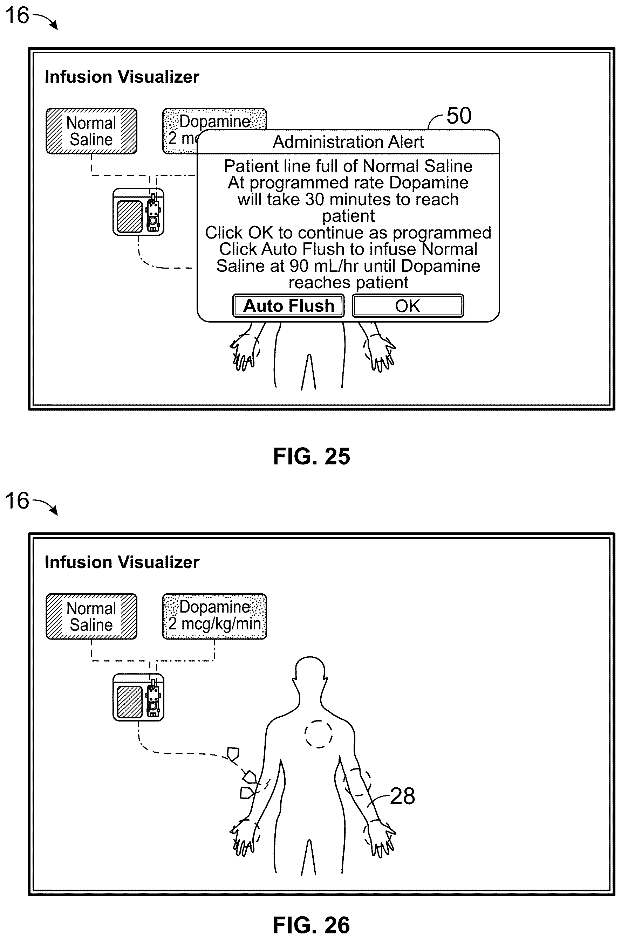

Upon the second infusion being added to the procedure, the controller 14 analyzes the procedure to determine (among other things) when the second fluid would reach the subject. In Fig. 25, the controller 14 has determined when the second fluid would reach the subject and provides a corresponding input to the display screen 16, which responds to the input by presenting a notice 50 indicating the time it would take for the second fluid to reach the subject. The illustrated notice 50 gives the operator the option of proceeding with the procedure as currently constituted or adjusting an operational parameter of the procedure (e.g., the rate at which the first fluid is delivered to the subject) so as to adjust the time it would take for the second fluid to reach the subject.

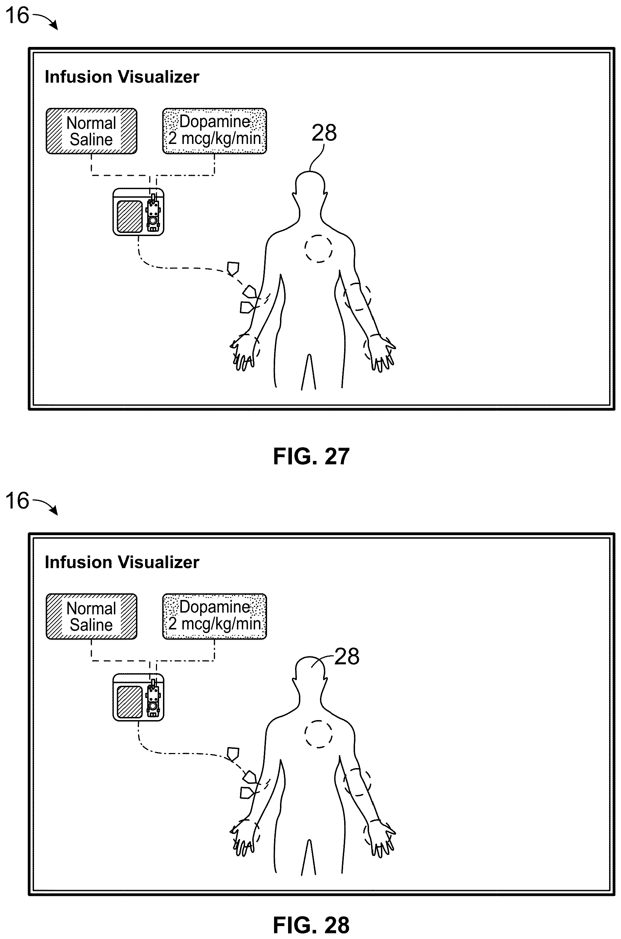

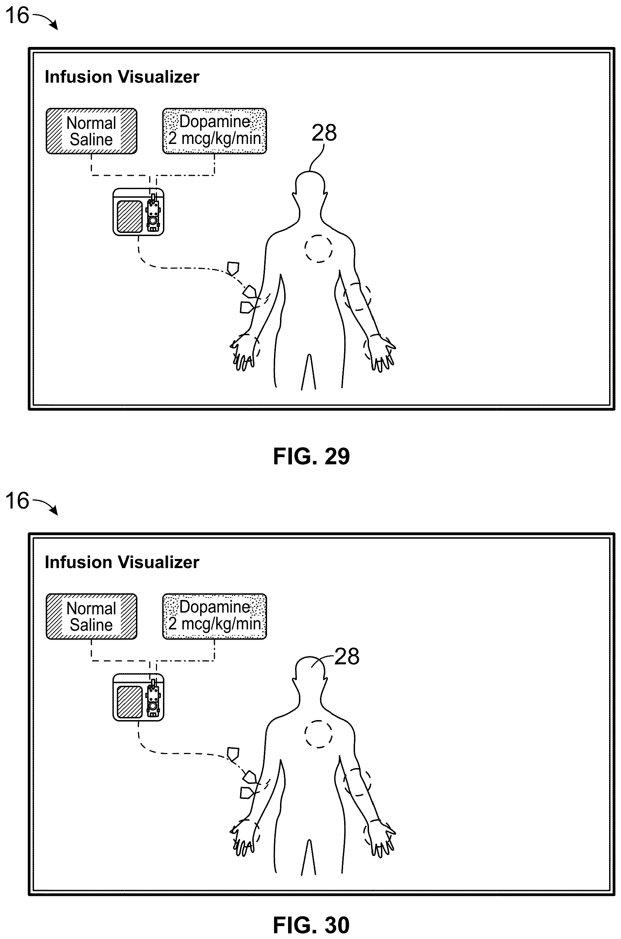

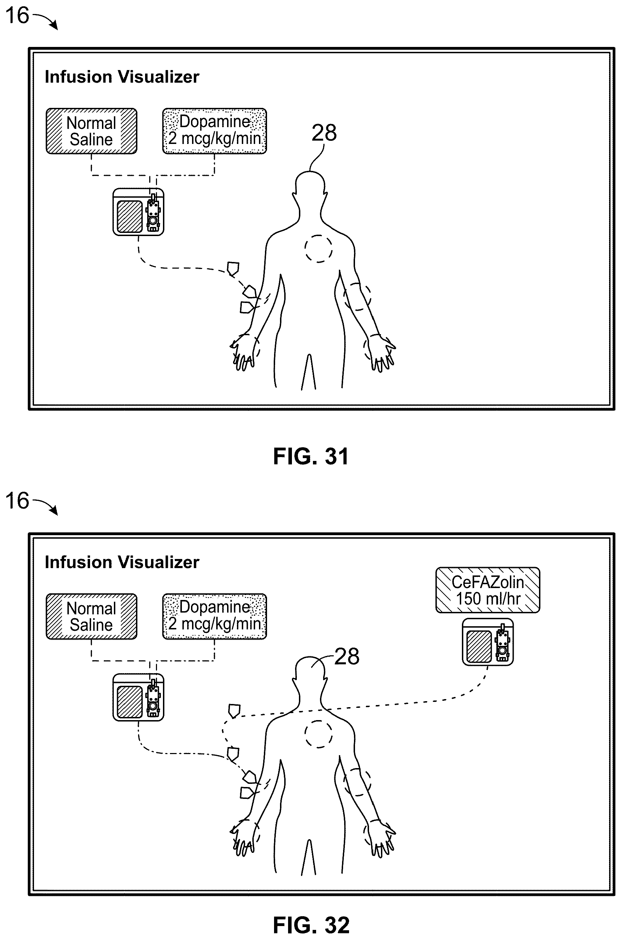

Figs. 26-31 visually represent the positions of the two fluids at different times during the proposed intravenous infusion procedure. In Fig. 26, the second fluid is first pumped into a conduit of an infusion set by the pump, with the first fluid in the conduit downstream of the second fluid (i.e., between the second fluid and the subject). In Fig. 27, an additional amount of the second fluid has been pumped into the conduit by the pump, with the second fluid reaching a point approximately halfway between the pump and the subject. In Fig. 28, the second fluid has reached the subject and the pump continues pumping the second fluid from the source container to the subject until the target volume of the second fluid has been delivered to the subject. Upon the target volume of the second fluid being delivered to the subject (or at least present in the conduit of the infusion set), the pump proceeds to convey the first fluid into the conduit from its source container, with Fig. 29 showing the first fluid in the conduit, upstream of the second fluid. Finally, Figs. 30 and 31 show the second fluid nearly entirely delivered to the subject (Fig. 30) and then fully delivered to the subject (Fig. 31), with only the first fluid present in the conduit.

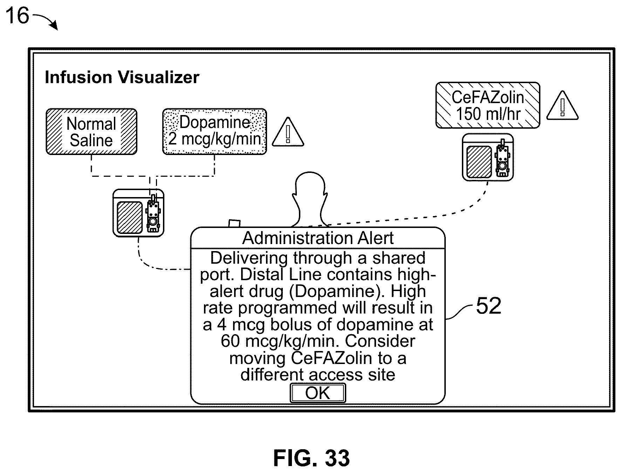

Fig. 32 shows a variation of the procedure that is simulated in Figs. 23-31, with a third fluid (which is identified in Fig. 32 as a dotted line) and second pump being added to the procedure at the same access site as the first pump and using a portion of the conduit of the same infusion set. Fig. 32 shows the third fluid at a Y-site, while the second fluid is being delivered to the subject via the infusion set (as in the condition shown in Fig. 28). The controller 14 analyzes the proposed procedure and, in Fig. 33, provides the display screen with input to display a bolus notice 52. The bolus notice 52 informs the operator of a possible negative interaction in which the proposed flow of the third fluid may result in bolusing of the second fluid, with the notice 52 suggesting that the third fluid be delivered to the subject at a different access site. It should be understood that the illustrated bolus notice 52 is merely exemplary and that such a notice may be differently configured without departing from the scope of the present disclosure.

The notices described above are merely exemplary, and additional notices may be presented by the display screen 16 in response to input from the controller 14 without departing from the scope of the present disclosure. For example, upon a second fluid being added to a proposed procedure, the controller 14 may determine whether a negative interaction could result from the compositions of the fluids (e.g., an incompatibility between the two fluids whereby the presence of one fluid affects the efficacy of the other fluid) and provide input to the display screen 16 to generate an appropriate notice, which may include a suggested approach for avoiding the negative interaction (such as replacing one of the fluids with a different fluid that would not create the risk of a negative interaction).

In view of the foregoing, it will be seen that intravenous infusion systems according to the present disclosure promote safer infusions for patients (by predicting possible negative interactions and optionally suggesting approaches to avoiding such interactions), while also producing more efficient procedures (due to fewer alerts and stoppages arising during a procedure). Intravenous infusion systems according to the present disclosure may also improve procedural efficiency in other ways, such as by enabling dead volume infusion across pumps.

### Aspects

Aspect 1. An intravenous infusion system, comprising: a plurality of infusion pumps each configured to convey a fluid to a subject during an intravenous infusion procedure; a controller programmed to receive information regarding each one of said infusion pumps; and a display screen configured to receive input from the controller and to display a visual representation of each one of said infusion pumps based at least in part on said input from the controller, wherein the controller is further programmed to determine whether there is a possible negative interaction between operation of at least two of said infusion pumps during the intravenous infusion procedure, control the display screen to display a notice regarding the negative interaction upon determining that the negative interaction is possible, and control the display screen to not display said notice upon determining that the negative interaction is not possible.

Aspect 2. The intravenous infusion system of Aspect 1, wherein the controller is programmed to receive said information from at least one of the infusion pumps.

Aspect 3. The intravenous infusion system of any one of the preceding Aspects, wherein the controller is programmed to receive said information from an operator.

Aspect 4. The intravenous infusion system of Aspect 3, wherein the display screen is configured to receive said information from the operator and to transmit said information to the controller.

Aspect 5. The intravenous infusion system of any one of the preceding Aspects, wherein the controller is programmed to determine whether the negative interaction is possible based at least in part on a location of said at least two of said infusion pumps.

Aspect 6. The intravenous infusion system of any one of the preceding Aspects, wherein each one of the infusion pumps includes an associated infusion set in fluidic communication with the vasculature of the subject, and the controller is programmed to determine whether the negative interaction is possible based at least in part on a characteristic of the infusion sets associated with said at least two of said infusion pumps.

Aspect 7. The intravenous infusion system of any one of the preceding Aspects, wherein the controller is programmed to determine whether the negative interaction is possible based at least in part on a composition of each of the fluids to be conveyed to the subject by said at least two of said infusion pumps.

Aspect 8. The intravenous infusion system of any one of the preceding Aspects, wherein each one of the infusion pumps includes an associated infusion set connected to the vasculature of the subject at a location, and the controller is programmed to determine whether the negative interaction is possible based at least in part on the locations at which the infusion sets associated with said at least two of said infusion pumps are connected to the vasculature of the subject.

Aspect 9. The intravenous infusion system of any one of the preceding Aspects, wherein the controller is programmed to determine whether the negative interaction is possible based at least in part on a rate at which each of the fluids is to be conveyed to the subject by said at least two of said infusion pumps.

Aspect 10. The intravenous infusion system of any one of the preceding Aspects, wherein the controller is programmed to determine a total fluid intake level and to control the display screen to display the total fluid intake level.

Aspect 11. The intravenous infusion system of any one of the preceding Aspects, wherein the display screen is configured to receive input from an operator to control operation of at least one of said infusion pumps.

Aspect 12. The intravenous infusion system of any one of the preceding Aspects, further comprising a bar code reader associated with the controller.

Aspect 13. The intravenous infusion system of any one of the preceding Aspects, further comprising a pump detector associated to the controller and configured to automatically detect a pump located in the vicinity of the pump detector.

Aspect 14. The intravenous infusion system of any one of the preceding Aspects, wherein the controller is programmed to receive information regarding an identity of the subject and to determine the infusion pumps to be visually represented on the display screen based on the identity of the subject.

Aspect 15. The intravenous infusion system of any one of the preceding Aspects, wherein the controller is programmed to control the display screen to display a visual representation of the subject and a location at which each pump is to convey fluid to the subject during the intravenous infusion procedure.

Aspect 16. The intravenous infusion system of any one of the preceding Aspects, wherein the controller is programmed to control the controller to display information regarding locations of the fluids during the intravenous infusion procedure.

Aspect 17. An intravenous infusion system, comprising: a plurality of infusion pumps each configured to convey a fluid to a subject during an intravenous infusion procedure; a controller programmed to receive information regarding each one of said infusion pumps; and a display screen configured to receive input from the controller and to display a visual representation of each one of said infusion pumps based at least in part on said input from the controller, wherein the controller is further programmed to control the display screen to display information regarding locations of the fluids during the intravenous infusion procedure.

Aspect 18. The intravenous infusion system of Aspect 17, wherein the controller is programmed to receive said information from at least one of the infusion pumps.

Aspect 19. The intravenous infusion system of any one of Aspects 17-18, wherein the controller is programmed to receive said information from an operator.

Aspect 20. The intravenous infusion system of Aspect 19, wherein the display screen is configured to receive said information from the operator and to transmit said information to the controller.

Aspect 21. The intravenous infusion system of any one of Aspects 17-20, wherein the controller is programmed to determine a total fluid intake level and to control the display screen to display the total fluid intake level.

Aspect 22. The intravenous infusion system of any one of Aspects 17-21, wherein the display screen is configured to receive input from an operator to control operation of at least one of said infusion pumps.

Aspect 23. The intravenous infusion system of any one of Aspects 17-22, further comprising a bar code reader associated with the controller.

Aspect 24. The intravenous infusion system of any one of Aspects 17-23, further comprising a pump detector associated to the controller and configured to automatically detect a pump located in the vicinity of the pump detector.

Aspect 25. The intravenous infusion system of any one of Aspects 17-24, wherein the controller is programmed to receive information regarding an identity of the subject and to determine the infusion pumps to be visually represented on the display screen based on the identity of the subject.

Aspect 26. The intravenous infusion system of any one of Aspects 17-25, wherein the controller is programmed to control the display screen to display a visual representation of the subject and a location at which each pump is to convey fluid to the subject during the intravenous infusion procedure.

Aspect 27. An intravenous infusion system, comprising: a plurality of infusion pumps each configured to convey a fluid to a subject during an intravenous infusion procedure; a controller programmed to receive information regarding each one of said infusion pumps; and a display screen configured to receive input from the controller and to display a visual representation of each one of said infusion pumps based at least in part on said input from the controller, wherein the controller is further programmed to determine a total fluid intake level and to control the display screen to display the total fluid intake level.

Aspect 28. The intravenous infusion system of Aspect 27, wherein the controller is programmed to receive said information from at least one of the infusion pumps.

Aspect 29. The intravenous infusion system of any one of Aspects 27-28, wherein the controller is programmed to receive said information from an operator.

Aspect 30. The intravenous infusion system of Aspect 29, wherein the display screen is configured to receive said information from the operator and to transmit said information to the controller.

Aspect 31. The intravenous infusion system of any one of Aspects 27-30, wherein the display screen is configured to receive input from an operator to control operation of at least one of said infusion pumps.

Aspect 32. The intravenous infusion system of any one of Aspects 27-31, further comprising a bar code reader associated with the controller.

Aspect 33. The intravenous infusion system of any one of Aspects 27-32, further comprising a pump detector associated to the controller and configured to automatically detect a pump located in the vicinity of the pump detector.

Aspect 34. The intravenous infusion system of any one of Aspects 27-33, wherein the controller is programmed to receive information regarding an identity of the subject and to determine the infusion pumps to be visually represented on the display screen based on the identity of the subject.

Aspect 35. The intravenous infusion system of any one of Aspects 27-34, wherein the controller is programmed to control the display screen to display a visual representation of the subject and a location at which each pump is to convey fluid to the subject during the intravenous infusion procedure.

It will be understood that the embodiments described above are illustrative of some of the applications of the principles of the present subject matter. Numerous modifications may be made by those skilled in the art without departing from the spirit and scope of the claimed subject matter, including those combinations of features that are individually disclosed or claimed herein. For these reasons, the scope hereof is not limited to the above description but is as set forth in the following claims, and it is understood that claims may be directed to the features hereof, including as combinations of features that are individually disclosed or claimed herein.

## Claims

1. An intravenous infusion system (10), comprising:
a plurality of infusion pumps (12) each configured to convey a fluid to a subject during an intravenous infusion procedure;
a controller (14) programmed to receive information regarding each one of said infusion pumps (12); and
a display screen (16) configured to receive input from the controller (14) and to display a visual representation of each one of said infusion pumps (12) based at least in part on said input from the controller (14), wherein
the controller (14) is further programmed to control the display screen (16) to display information regarding locations of the fluids during the intravenous infusion procedure, or
the controller (14) is further programmed to determine a total fluid intake level and to control the display screen (16) to display the total fluid intake level, or
the controller (14) is further programmed to
determine whether there is a possible negative interaction between operation of at least two of said infusion pumps (12) during the intravenous infusion procedure,
control the display screen (16) to display a notice regarding the negative interaction upon determining that the negative interaction is possible, and
control the display screen (16) to not display said notice upon determining that the negative interaction is not possible.

2. The intravenous infusion system (10) of claim 1, wherein the controller (14) is programmed to receive said information from at least one of the infusion pumps (12).

3. The intravenous infusion system (10) of any one of the preceding claims, wherein the controller (14) is programmed to receive said information from an operator.

4. The intravenous infusion system (10) of claim 3, wherein the display screen (16) is configured to receive said information from the operator and to transmit said information to the controller (14).

5. The intravenous infusion system (10) of any one of the preceding claims, wherein the controller (14) is programmed to determine whether the negative interaction is possible based at least in part on a location of said at least two of said infusion pumps (12).

6. The intravenous infusion system (10) of any one of the preceding claims, wherein
each one of the infusion pumps (12) includes an associated infusion set (18) in fluidic communication with the vasculature of the subject, and
the controller (14) is programmed to determine whether the negative interaction is possible based at least in part on a characteristic of the infusion sets (18) associated with said at least two of said infusion pumps (12).

7. The intravenous infusion system (10) of any one of the preceding claims, wherein the controller (14) is programmed to determine whether the negative interaction is possible based at least in part on a composition of each of the fluids to be conveyed to the subject by said at least two of said infusion pumps (12).

8. The intravenous infusion system (10) of any one of the preceding claims, wherein
each one of the infusion pumps (12) includes an associated infusion set (18) connected to the vasculature of the subject at a location, and
the controller (14) is programmed to determine whether the negative interaction is possible based at least in part on the locations at which the infusion sets (18) associated with said at least two of said infusion pumps (12) are connected to the vasculature of the subject.

9. The intravenous infusion system (10) of any one of the preceding claims, wherein the controller (14) is programmed to determine whether the negative interaction is possible based at least in part on a rate at which each of the fluids is to be conveyed to the subject by said at least two of said infusion pumps (12).

10. The intravenous infusion system (10) of any one of the preceding claims, wherein the display screen (16) is configured to receive input from an operator to control operation of at least one of said infusion pumps (12).

11. The intravenous infusion system (10) of any one of the preceding claims, further comprising a bar code reader (20) associated with the controller (14).

12. The intravenous infusion system (10) of any one of the preceding claims, further comprising a pump detector (22) associated to the controller (14) and configured to automatically detect a pump (12) located in the vicinity of the pump detector (22).

13. The intravenous infusion system (10) of any one of the preceding claims, wherein the controller (14) is programmed to receive information regarding an identity of the subject and to determine the infusion pumps (12) to be visually represented on the display screen (16) based on the identity of the subject.

14. The intravenous infusion system (10) of any one of the preceding claims, wherein the controller (14) is programmed to control the display screen (16) to display a visual representation of the subject and a location at which each pump (12) is to convey fluid to the subject during the intravenous infusion procedure.
